# EUROPÄISCHE PATENTSCHRIFT

(11) **EP 1 915 212 B1**
(45) Veröffentlichungstag und Bekanntmachung des Hinweises auf die Patenterteilung: **09.02.2011**
(21) Anmeldenummer: 06806069.8
(22) Anmeldetag: 05.10.2006
(51) Int. Cl.: B01L 3/00

(54) **VORRICHTUNG UND VERFAHREN ZUR BESTIMMUNG DER VOLUMENANTEILE DER PHASEN IN EINER SUSPENSION**
DEVICE AND METHOD FOR DETERMINING THE VOLUME PARTS OF PHASES IN A SUSPENSION
DISPOSITIF ET PROCEDE POUR DETERMINER LES FRACTIONS VOLUMIQUES DES PHASES D'UNE SUSPENSION

(30) Priorität: 07.10.2005 DE 102005048236
(43) Veröffentlichungstag der Anmeldung: 30.04.2008
(73) Patentinhaber: Albert-Ludwigs-Universität Freiburg, 79098 Freiburg (DE)
(72) Erfinder: GRUMANN, Markus, 37085 Göttingen (DE); DUCRÉE, Jens, 79112 Freiburg (DE); ZENGERLE, Roland, 79183 Waldkirch (DE); RIEGGER, Lutz, 79108 Freiburg (DE)
(74) Vertreter: Zinkler, Franz
(86) Internationale Anmeldenummer: PCT/EP2006/009660
(87) Internationale Veröffentlichungsnummer: WO 2007/042207

(56) Entgegenhaltungen:
- WO-A-00/53321
- WO-A-98/39645
- WO-A-99/41147
- US-A- 4 740 472
- US-A- 4 761 381

## Beschreibung

Die vorliegende Anmeldung bezieht sich auf eine Vorrichtung und ein Verfahren zur Bestimmung der Volumenanteile der Phasen in einer Suspension, d. h. einem Mehrphasengemisch, das eine flüssige Phase und eine feste Phase enthält. Insbesondere eignet sich die vorliegende Erfindung zur Bestimmung des Hämatokritwertes HKT von Vollblut, d. h. des Verhältnisses des Teilvolumens der zellulären Bestandteile zum Gesamtvolumen.

Aus dem Stand der Technik sind Verfahren zur Ermittlung des Hämatokritwertes HKT von Blut bekannt. Ein bekanntes Verfahren zum Ermitteln des Hämatokritwertes basiert auf einer elektrischen Leitwertmessung, bei der der gemessene Leitwert im umgekehrten Verhältnis proportional zum Hämatokrit ist. Derartige Verfahren sind beispielsweise in "Labor und Diagnose" von Lothar Thomas, TH-Books, 5. Auflage, 1998, und K. Dörner, "Klinische Chemie und Hämatologie", Georg Thieme Verlag, Stuttgart, Deutschland, 1989, 2003, beschrieben. Darüber hinaus wurden zum Anmeldezeitpunkt von der Firma iSTAT Corporation, East Windsor, NJ, USA (http://www.istat.com) Produkte zur Hämatokrit-Bestimmung unter Verwendung eine elektrischen Leitwertmessung angeboten.

Ein weiteres Verfahren zur Bestimmung des Hämatokritwertes wird als Mikrohämatokrit-Methode bezeichnet. Dabei wird eine Mikrokapillare mit einem Innendurchmesser von 1 mm in das zu messende Blut getaucht. Das Blut steigt, angetrieben durch die kapillare Kraft, in der Kapillare auf. Diese wird nun an einem Ende versiegelt und in eine Mikrohämatokritzentrifuge oder einen Mikrohämatokritrotor eingesetzt und nach dem NCCLS-Standard zentrifugiert. Die Bestimmung des Hämatokritwertes HKT erfolgt entweder durch eine Messscheibe oder eine Messharfe. Mit der Messscheibe ist ein direktes Auslesen des Hämatokritwertes noch in der Zentrifuge möglich. Der große Nachteil dieser Methode liegt in der notwendigen manuellen Versiegelung der Kapillare.

Die Mikrohämatokrit-Methode ist als Referenzmethode anerkannt, wobei die erhaltenen Werte aufgrund des eingeschlossenen Plasmas bis zu etwa 2 % höher liegen als die Vergleichsmessung mit einem Hämatologieanalysator. Hinsichtlich dieser Mikrohämatokrit-Methode kann beispielsweise auf K. Dörner, Klinische Chemie und Hämatologie, Georg Thieme Verlag, Stuttgart, Deutschland, 1989, 2003, oder B. Bull et al., Pennsylvania, USA, ISBN 1-56238-413-9 (1994) verwiesen werden. Ferner wird diese Technik zum Anmeldezeitpunkt von der Firma Hermle Labortechnik GmbH (http://www.hermle-labortechnik.de) praktiziert.

Aus dem Stand der Technik sind ferner Verfahren zum Befüllen von Blindkanälen, d. h. Kanälen mit einem geschlossenen Ende, bekannt, die einen Einschluss von Blasen verhindern sollen. Derartige Verfahren sind beispielsweise bei Steinert CP Sandmaier H, Daub M., de Heij B., Zengerle R. (2004), Bubble free priming of blind channels, in Proceedings of IEEE-MEMS, 25. - 29. Januar 2004, Maastricht, The Netherlands, S. 224 - 228; und Goldschmidtboeing F., Woias P. (2005), Strategies for Void-free Liquid-filling of Micro Cavities, in Proceedings of Transducers '05 Conference, 05. - 09. Juni, Seoul, Korea, ISBN 07-7803-8994-8, S. 1561 - 1564; sowie in der DE 10325110 B3 beschrieben.

Die Aufgabe der vorliegenden Erfindung besteht darin, ein neuartiges Konzept zur Bestimmung der Volumenanteile der Phasen in einer Suspension zu schaffen, das zuverlässige Ergebnisse unter Verwendung geringer Suspensionsvolumina ermöglicht.

Diese Aufgabe wird durch eine Vorrichtung nach Anspruch 1 sowie ein Verfahren nach Anspruch 13 gelöst.

Die vorliegende Erfindung schafft eine Vorrichtung zur Bestimmung der Volumenanteile der Phasen in einer Suspension mit folgenden Merkmalen:
einem Körper;
einer Kanalstruktur, die in dem Körper gebildet ist und einen Einlassbereich und einen Blindkanal, der mit dem Einlassbereich fluidisch verbunden und über denselben befüllbar ist, aufweist; und
einer Antriebseinrichtung zum Beaufschlagen des Körpers mit einer Rotation, so dass durch eine Zentrifugation eine Phasentrennung der Suspension in dem Blindkanal stattfindet,
wobei der Blindkanal einen solchen Kanalquerschnitt und/oder solche Benetzungseigenschaften aufweist, dass beim Befüllen desselben über den Einlassbereich mit der Suspension in einem ersten Querschnittbereich höhere Kapillarkräfte als in einem zweiten Querschnittbereich wirken, so dass sich zunächst der erste Querschnittbereich in Richtung von dem Einlassbereich zu dem blinden Ende hin füllt und dann der zweite Querschnittbereich in Richtung von dem blinden Ende zu dem Einlassbereich hin füllt.

Die vorliegende Erfindung schafft ferner ein Verfahren zur Bestimmung der Volumenanteile der Phasen in einer Suspension mit folgenden Schritten:
Bereitstellen einer Kanalstruktur, die einen Einlassbereich und einen an den Einlassbereich angrenzenden Blindkanal aufweist;
Einbringen der Suspension in den Einlassbereich, wobei der Blindkanal einen solchen Kanalquerschnitt und/oder solche Benetzungseigenschaften aufweist, dass in einem ersten Querschnittbereich höhere Kapillarkräfte als in einem zweiten Querschnittbereich wirken, sodass sich zunächst der erste Querschnittbereich in Richtung von dem Einlassbereich zu dem blinden Ende hin füllt und dann der zweite Querschnittbereich in Richtung von dem blinden Ende zu dem Einlassbereich hin füllt, wenn die Suspension in den Einlassbereich eingebracht wird; und
Beaufschlagen der Kanalstruktur mit einer Rotation, um eine Phasentrennung der Suspension in dem Blindkanal durch Zentrifugation zu bewirken.

Die vorliegende Erfindung bezieht sich auf ein neuartiges Konzept, um die Volumenanteile der Phasen in einem Mehrphasengemisch zu bestimmen. Das erfindungsgemäße Konzept nutzt dabei den Effekt einer Sedimentation in einem Blindkanal, wenn derselbe einer Zentrifugation unterworfen wird. Der Blindkanal umfasst erfindungsgemäß einen solchen Kanalquerschnitt und/oder solche Benetzungseigenschaften, dass eine asymmetrische Kapillarkraft entlang der Wände des Blindkanals auftritt, was ein kapillares Befüllen des Kanals bevorzugt im Bereich der hohen Kapillarkräfte zur Folge hat. Dadurch wird Luft in den Bereich der niedrigen Kapillarkraft hinein verdrängt und darüber hinaus in Richtung des Einlasses. Somit wird durch eine schnelle Befüllrate in dem Bereich der hohen Kapillarkräfte der zugeordnete Querschnittbereich des Kanals schnell in Richtung von der offenen Seite zu der geschlossenen Seite hin gefüllt, woraufhin danach die Bereiche mit der niedrigen Kapillarekraft in der Richtung von dem blinden Ende zu dem Einlass hin befüllt werden. Dies ermöglicht eine Befüllung des Blindkanals im Wesentlichen ohne Lufteinschluss. Der Blindkanal ist somit aufgrund des Kanalquerschnitts und/oder der Benetzungseigenschaften mit der Probe mit einem definierten und in der Regel verschwindenden Blaseneinschluss befüllbar. Der Blindkanal wird einer Zentrifugation unterworfen, sodass eine Phasentrennung der Suspension erfolgt und die Partikel aus der Suspension heraussedimentiert werden.

Bei bevorzugten Ausführungsbeispielen kann die Kanalstruktur eine integrierte Überlaufstruktur zwischen Einlass und Blindkanal zur integrierten Volumendefinition der Probe aufweisen. Bei weiteren Ausführungsbeispielen kann eine Skala zur Ablesung der Volumenanteile in dem Körper, in dem die Kanalstruktur gebildet ist, integriert sein. Der Körper, in dem die Kanalstruktur gebildet ist, kann bei Ausführungsbeispielen der vorliegenden Erfindung durch eine erste Schicht, in der die Kanalstruktur gebildet ist, und eine zweite Schicht, die einen Deckel bildet, gebildet sein.

Um asymmetrische Kapillarkräfte entlang der Wände des Blindkanals zu bewirken, kann der Blindkanal Wände aufweisen, die mit unterschiedlichen eingeschlossenen Winkeln aneinandergrenzen. Zusätzlich oder alternativ können die Wände bezüglich der Suspension unterschiedlich hydrophil sein oder Abschnitte aufweisen, die bezüglich der Suspension unterschiedlich hydrophil sind. Wiederum alternativ oder zusätzlich kann der Blindkanal einen Querschnitt mit zumindest einer Stufe aufweisen, so dass sich eine Kapillarkraftverteilung über den Querschnitt des Blindkanals ergibt, die Bereiche mit höherer Kapillarkraft und Bereiche mit geringerer Kapillarkraft aufweist.

Beim erfindungsgemäßen Verfahren zur Bestimmung der Volumenanteile der Phasen in einer Suspension kann darüber hinaus die Zentrifugalkraft ausgenutzt werden, um ein beschleunigtes Befüllen des Blindkanals zu bewirken. Zu diesem Zweck kann bereits eine Rotation der Kanalstruktur bewirkt werden, bevor der Blindkanal vollständig befüllt ist.

Die vorliegende Erfindung ermöglicht eine vollständige Integration aller für eine Hämatokritwert-Bestimmung notwendigen Prozessschritte, wobei insbesondere kein nachträgliches Versiegeln einer Kapillare notwendig ist. Ferner lässt sich die erfindungsgemäße Vorrichtung über einen einfachen Prozess herstellen, da der Körper auf einfache Weise aus zwei Schichten bestehen kann, wobei in einer derselben die Kanalstruktur strukturiert ist, während die andere als Deckel dient. Alternativ können beide Schichten strukturiert sein, um Teile der Kanalstruktur zu definieren.

Die vorliegende Erfindung kann als sogenanntes "Lab-on-a-Disk"-System implementiert werden, wobei auf dem Körper weitere medizinische Tests integriert werden können, die ebenfalls Zentrifugal- und Kapillarkräfte sowie weitere in sogenannten Lab-on-a-Chip-Systemen übliche Kräfte ausnutzen. Die vorliegende Erfindung eignet sich insbesondere zur Bestimmung des Hämatokritwertes von Blut, wobei entsprechend die Abmessungen der Kanalstruktur angepasst sind, um eine Sedimentation des Bluts in rote Blutkörperchen und Plasma in dem Blindkanal bewirken zu können. Lab-on-a-Chip-Systeme sind beispielsweise bei A. van den Berg, E. Oosterbroek, Amsterdam, NL, ISBN 0-444-51100-8 (2003), beschrieben.

Der Blindkanal ist für eine kapillare Befüllung mit der Suspension, deren Volumenanteile bestimmt werden sollen, ausgelegt, wobei das Befüllen somit ohne Zentrifugalkraft stattfinden kann. Die Zentrifugalkraft kann jedoch unterstützend verwendet werden, um den Befüllungsvorgang zu beschleunigen, indem die Kanalstruktur während der Befüllung mit einer Rotation beaufschlagt wird.

Bevorzugte Ausführungsbeispiele der vorliegenden Erfindung werden nachfolgend Bezug nehmend auf die beiliegenden Zeichnungen näher erläutert. Es zeigen:
- Fig. 1: eine schematische Darstellung eines Substrats gemäß einem Ausführungsbeispiel der Erfindung;
- Fig. 2: eine schematische Querschnittsdarstellung eines Kanals zur Erläuterung einer asymmetrischen Ka- pillardruckverteilung;
- Fig. 3A: bis 3 F schematisch Kanalquerschnitte, wie sie bei Ausführungsbeispielen der Erfindung verwendet werden können;
- Fig. 4A: und 4B schematisch einen jeweiligen Abschnitt eine Kanalquerschnitts zur Erläuterung der Erzeu- gung eines asymmetrischen Kanaldrucks unter Ver- wendung unterschiedlicher Benetzungswinkel;
- Fig. 5: eine schematische perspektivische Ansicht des blinden Endes eines Ausführungsbeispiels eines Blindkanals, dessen Querschnitt in Fig. 3A ge- zeigt ist;
- Fig. 6A: bis 6C Seitenansichten des in Fig. 5 gezeigten Kanals in unterschiedlichen Phasen der Befüllung desselben;
- Fig. 6D: bis 6F Draufsichten des Kanals von Fig. 5, eben- falls in unterschiedlichen Phasen der Befüllung desselben, die den Phasen der Fig. 6A bis 6C ent- sprechen;
- Fig. 7A: bis 7C eine Kanalstruktur zu unterschiedlichen Zeitpunkten eines Ausführungsbeispiels des erfin- dungsgemäßen Verfahrens;
- Fig. 8: ein Frequenzprotokoll zur Steuerung einer An- triebseinrichtung während der Durchführung eines Ausführungsbeispiels des erfindungsgemäßen Ver- fahrens;
- Fig. 9: schematisch das Ergebnis einer Messreihe zur Hämatokritbestimmung unter Verwendung einer Ka- nalstruktur, wie sie in Fig. 7 gezeigt ist;
- Fig. 10A und 10B: schematische Draufsichten von Ausfüh- rungsbeispielen eines als Scheibe ausgebildeten Substrats; und
- Fig. 11: eine schematische Seitenansicht eines Ausfüh- rungsbeispiels einer erfindungsgemäßen Vorrich- tung.

Die vorliegende Erfindung eignet sich allgemein zur Bestimmung der Volumenanteile der Phasen in einem Mehrphasengemisch, und ist insbesondere zur Bestimmung des Hämatokritwertes von Blut vorteilhaft einsetzbar.

Die vorliegende Erfindung umfasst im Wesentlichen einen Körper und eine Antriebseinrichtung zum Beaufschlagen des Körpers mit einer Rotation. Der Körper kann beispielsweise ein gedeckeltes Substrat, in dem Kanalstrukturen implementiert sind, aufweisen und über einen Drehmotor in eine Rotationsbewegung versetzt werden. Dabei kann der Körper entweder selbst als ein Rotationskörper, beispielsweise eine Scheibe ausgebildet sein, der auf eine geeignete Kupplung des Drehmotors gesetzt wird, oder der Körper kann als ein Modul ausgelegt sein, dass in einen Rotor eingelegt werden kann, der durch einen Drehmotor antreibbar ist. Für eine technische Realisation ist weniger die genaue Form des Körpers als vielmehr die Auswuchtung des Rotors von Bedeutung.

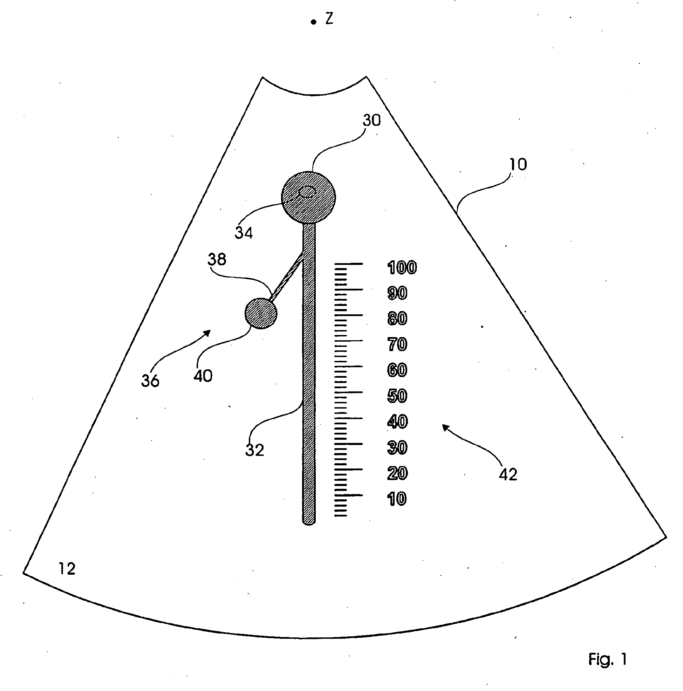

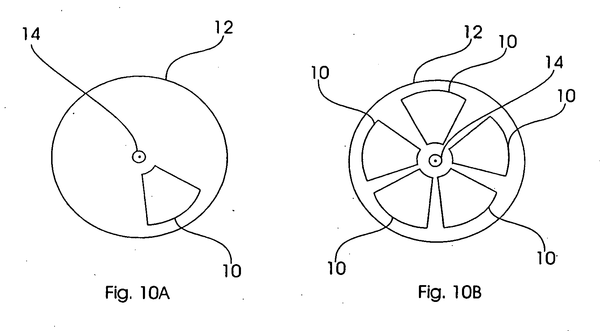

Fig. 1 zeigt eine schematische Draufsicht auf einen Ausschnitt 10 eines Substrats, das beispielsweise als eine Scheibe 12, wie sie in Fig. 10A gezeigt ist, implementiert sein kann. Das Substrat 12 kann nach Art einer herkömmlichen CD aufgebaut sein, mit einer mittleren Öffnung 14, mittels derer sie beispielsweise an einer herkömmlichen Zentrifuge angebracht werden kann. Ein alternatives Ausführungsbeispiel eines Substrats 12', in dem eine Mehrzahl von Kanalstrukturen gebildet sind, das somit eine Mehrzahl von Bereichen 10 aufweist, ist in Fig. 10B gezeigt. Durch das in Fig. 10B gezeigte Substrat, in dem fünf Kanalstrukturen gebildet sind, kann gleichzeitig oder auch nacheinander der Hämatokritwert von fünf Blutproben ermittelt werden.

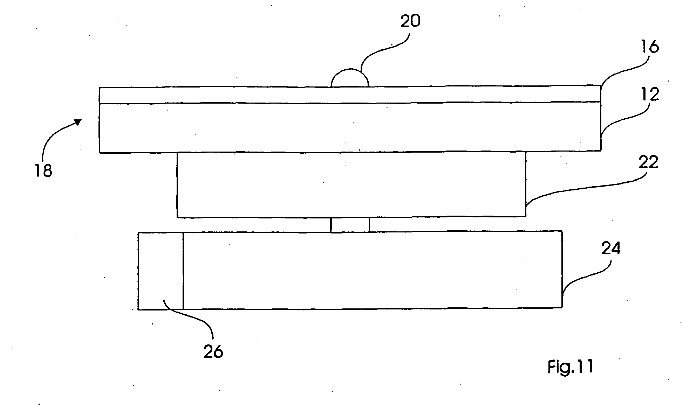

Wie der Fig. 11 zu entnehmen ist, ist das Substrat 12, in dem die Kanalstrukturen gebildet sind, mit einem Deckel 16 versehen. Das Substrat 12 und der Deckel 16 bilden einen Modulkörper 18. Der Modulkörper 18 ist über eine Befestigungseinrichtung 20 an einem rotierenden Teil 22 einer Antriebsvorrichtung angebracht, das drehbar an einem stationären Teil 24 der Antriebsvorrichtung gelagert ist. Bei der Antriebsvorrichtung kann es sich beispielsweise um eine herkömmliche Zentrifuge mit einstellbarer Drehgeschwindigkeit oder auch ein CD- oder DVD-Laufwerk handeln. Die Antriebsvorrichtung 24 umfasst eine Steuereinrichtung 26, um die jeweiligen Rotationen des Substrats 12, um das erfindungsgemäße Verfahren durchzuführen, zu bewirken.

Wie in Fig. 1 gezeigt ist, weist eine Kanalstruktur in dem Substrat einen Einlassbereich 30 für das zu untersuchende Medium auf, der an einen Blindkanal angrenzt. Das Substrat 12 ist um eine Rotationsachse Z drehbar, sodass der Einlassbereich radial nach außen in den Blindkanal 32 endet. In dem Einlassbereich befindet sich beispielsweise ein Loch 34 im Deckel des Substrats, wie in Fig. 1 durch gestrichelte Linien angedeutet ist. Durch das Loch kann eine Probe in den Einlassbereich eingeführt werden.

Die Kanalstruktur umfasst bei dem gezeigten Beispiel ferner eine Überlaufstruktur 36, die einen Überlaufkanal 38 und eine Überlaufkammer 40, in die der Überlaufkanal 38 mündet, aufweist. Die Überlaufstruktur 36 dient zur Volumendosierung der Probe, d. h. der Suspension. Der Überlaufkanal 38 der Überlaufstruktur kann eine hydrophobe Barriere zur Dosierung darstellen, die nach dem Befüllen des Blindkanals 32 überwunden wird, so dass sich ein definiertes Volumen der Suspension in dem Blindkanal 32 befindet.

Ferner umfasst bei dem gezeigten Ausführungsbeispiel das Substrat 12 eine Skala 42, die beispielsweise auf oder in dem Deckel oder auf der Oberseite der Trägerschicht 16 gebildet sein kann. Die Skala 42 ermöglicht ein direktes optisches Ablesen des Volumens des Phasenanteils nach der Sedimentation.

Der Blindkanal 32 ist derart ausgebildet, dass in unterschiedlichen Querschnittbereichen desselben unterschiedliche Kapillarkräfte wirken. Insbesondere kann der Blindkanal ausgebildet sein, um einen unterschiedlich starke Kapillarkräfte entlang der Kanten des Kanals zu erhalten. Zu diesem Zweck kann ein Neigungswinkel der Seitenwände des Kanals gegenüber einer zu den Hauptoberflächen des Substrats Senkrechten und/oder der Kontaktwinkel der Kanalinnenwand mit der zu sedimentierenden Suspension angepasst werden. Insbesondere können dadurch Zonen mit erhöhtem Kapillardruck erzeugt werden, wobei die Ausbreitung der Menisken dann mit der größten Geschwindigkeit entlang der Zonen mit dem erhöhten Kapillardruck stattfindet.

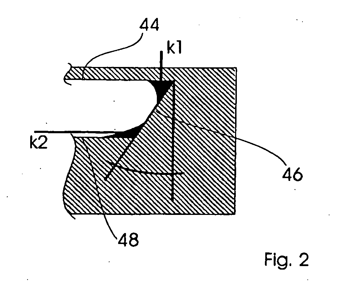

Gemäß einer ersten Alternative, wie sie schematisch in Fig. 2 gezeigt ist, können die Wände des Blindkanals bzw. die Wände der gesamten Kanalstruktur (Einlass und Blindkanal) um einen Winkel α geneigt sein. Durch eine solche Neigung α ergibt sich ein unterschiedlich hoher Kapillardruck an Kanten k1 und k2 des Kanals, wobei an der Kante k1 eine Seitenwand 46 und eine obere Wand 44 mit einem kleineren eingeschlossenen Winkel aneinandergrenzen als an der Kante k2 die Seitenwand 46 mit einer Kanalbodenwand 48. Somit herrscht im Bereich der Kante k1 eine höhere Kapillarkraft als im Bereich der Kante k2. Der zu der Kante k1 benachbarte Bereich stellt somit einen Bereich einer höheren Kapillarkraft dar, an dem eine Propagation des Meniskus einer Suspension, mit dem der Kanal befüllt werden soll, mit einer erhöhten Geschwindigkeit stattfindet. Somit kann erreicht werden, dass zunächst eine Befüllung in diesen Bereichen in Richtung von dem Einlassbereich zu dem blinden Ende hin stattfindet, und sich danach die übrigen Bereiche in Richtung von dem blinden Ende zu dem Einlassbereich hin füllen.

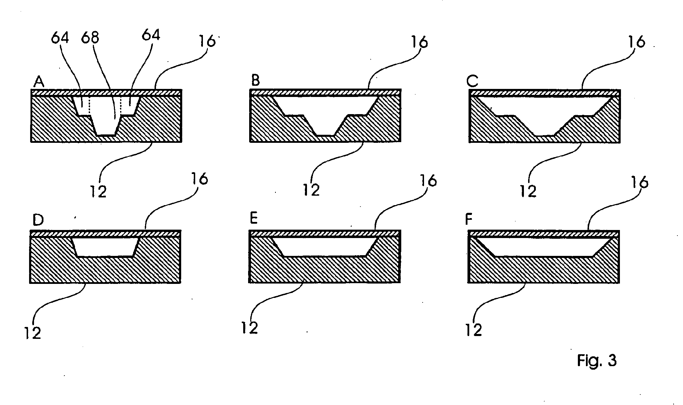

Variationen von Kanalquerschnitten sind in den Fig. 3A - 3F gezeigt, wobei der Kanal jeweils in dem Substrat 12, das mit einem Deckel 16 versehen ist, gebildet ist. In den Fig. 3A - 3C sind T-Kanalquerschnitte gezeigt, deren Seitenwände von Fig. 3A nach 3C zunehmend höhere Neigungswinkel zeigen. Ein erhöhter Neigungswinkel α einer bzw. mehrerer Kanalwände steigert die Asymmetrie des Kapillardrucks.

In den Fig. 3D - 3F sind trapezförmige Kanalquerschnitte gezeigt, deren Seitenwände von Fig. 3D nach 3F zunehmend höhere Neigungswinkel aufweisen und somit eine zunehmend höhere Asymmetrie der Kapillarkraft.

Die in den Fig. 3A - 3C gezeigten Kanalquerschnitte stellen dabei eine bevorzugte Ausführungsform dar, da dieselben ein zuverlässigeres blasenfreies Befüllen ermöglichen. Die beschriebenen Querschnitte sind vorteilhaft dahingehend, dass sie sich durch übliche Fräswerkzeuge technisch einfach herstellen lassen.

Alternativ zu den in Fig. 3A - 3C gezeigten "schrägen" T-Formen könnte der Kanalquerschnitt auch eine T-Form mit im Wesentlichen geraden Seitenflächen aufweisen, sodass der Kanal Stufen besitzt, die Querschnittbereiche definieren, in denen unterschiedliche Kapillarkräfte herrschen, sodass dadurch eine im Wesentlichen blasenfreie Befüllung möglich ist.

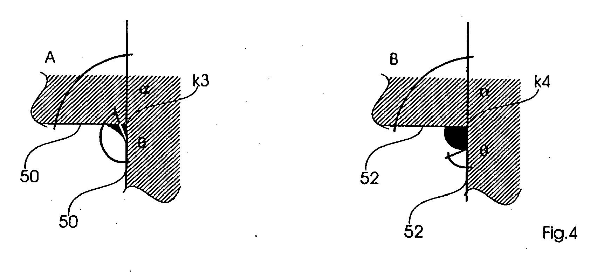

Als eine weitere Alternative lassen sich unterschiedlich starke Kapillardrücke in den Kanalkanten durch Variation des Kontaktwinkels θ realisieren. Diesbezüglich zeigt Fig. 4A schematisch eine Kante k3 eines Kanals, dessen Kanalwände bezüglich der zu befüllenden Suspension derart hydrophilisiert sind, dass ein großer Kontaktwinkel θ vorliegt. Dadurch ergibt sich im Bereich der Kante k3 eine hohe Kapillarkraft. Im Gegensatz dazu sind die Kanalwände an der in Fig. 4B gezeigten Kante k4 bezüglich der zu befüllenden Suspension derart hydrophilisiert, dass sich ein kleiner Kontaktwinkel θ ergibt. Dadurch herrscht im Bereich der Kante k4 ein geringerer Kontaktwinkel.

Gemäß Fig. 4A und 4B kann somit ein blasenfreies Befüllen des hydrophilen Blindkanals auch basierend auf dem bevorzugten kapillaren Befüllen entlang eines bestimmten Teils der Kanalwand durch Variation des Kontaktwinkels θ erfolgen, wobei der in Fig. 4A gezeigte Fall eine gegenüber dem in Fig. 4B gezeigten Fall bevorzugte kapillare Befüllung liefert. Ein erhöhter Neigungswinkel α der Kanalwand kann zusätzlich die Asymmetrie der Kapillarkraft steigern. Beispielsweise ist es denkbar, die Innenseite des Deckels 16 stärker zu hydrophilisieren als die Wände des Substrats 12, sodass dadurch eine an den Kanten zwischen Deckel 16 und Substrat 12 auftretender Kapillarkraft gegenüber einer in einem Bereich an den Kanten zwischen den Seitenwänden und Kanalboden auftretenden Kapillarkraft erhöht ist. Weiterhin können Wandabschnitte einzelner Wände stärker hydrophilisiert sein als andere, um dort Bereiche zu erzeugen, an denen eine höhere Kapillarkraft auftritt als in anderen Bereichen, um so die beschriebene Funktionalität zu erhalten.

Zusammenfassend kann festgestellt werden, dass die Kapillarkraft in unterschiedlichen Querschnittbereichen des Blindkanals durch die geometrischen Winkel und die Benetzungswinkel bestimmt wird, sodass durch eine entsprechende Konfiguration des Kanalquerschnitts unter Verwendung spitzer Winkel oder einer ausreichenden Hydrophilisierung der Effekt erreicht werden kann, dass der Blindkanal zunächst in Richtung von dem offenen Ende zu dem blinden Ende hin in bestimmten Bereichen befüllt wird und die übrigen Bereiche dann in Richtung von dem blinden bzw. geschlossenen Ende zu dem offenen Ende hin befüllt werden. Anders ausgedrückt findet in den Bereichen mit erhöhter Kapillarkraft eine Befüllung mit einer schnellen Befüllrate statt, während in den Bereichen mit einer geringen Kapillarkraft eine Befüllung mit einer langsamen Befüllrate stattfindet.

Hinsichtlich der Theorie einer derartigen blasenfreien Befüllbarkeit von Blindkanälen bzw. deren Ausgestaltung wird auf die eingangs genannten Schriften verwiesen, deren diesbezügliche Offenbarung durch Bezugnahme aufgenommen wird.

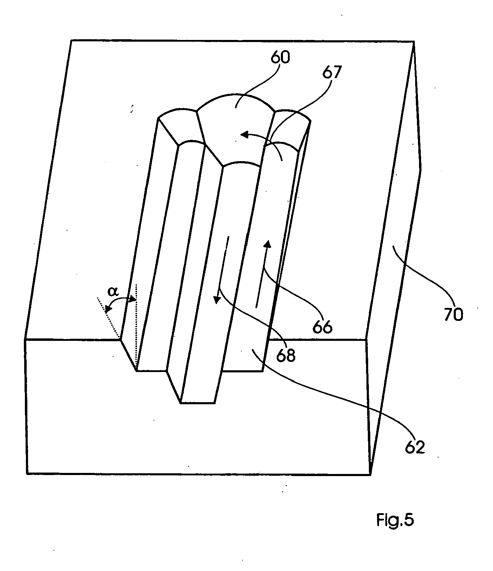

Eine perspektivische Ansicht einer Kanalstruktur, die einen Kanalquerschnitt, der im Wesentlichen dem in Fig. 3A gezeigten Querschnitt entspricht, ist in Fig. 5 gezeigt. Der Kanalquerschnitt besitzt eine T-Form, dessen Seitenwände einen Neigungswinkel α von etwa 17,5° aufweisen. An dem geschlossenen Ende 60 des Blindkanals, der allgemein mit dem Bezugszeichen 62 bezeichnet ist, befindet sich ein Übergangsbereich. Bei der in Fig. 5 gezeigten Kanalstruktur stellen die äußeren Bereiche des Querbalkens der T-Struktur, die in Fig. 3A schematisch markiert und mit dem Bezugszeichen 64 bezeichnet sind, Bereiche mit einem erhöhten Kapillardruck dar. Somit erfolgt eine Befüllung von der offenen Seite des Blindkanals 62 entlang dieser Bereiche zu dem geschlossenen Ende hin, wie durch einen Pfeil 66 in Fig. 5 gezeigt ist. An dem geschlossenen Ende 60 ist ein Übergang vorgesehen, um einen Übertritt der Suspension in den noch nicht befüllten inneren Bereich, der in Fig. 3A mit dem Bezugszeichen 68 bezeichnet ist, zu unterstützen. Dies ist in Fig. 5 durch einen Pfeil 67 angezeigt. Nachfolgend befüllt sich der Blindkanal weiter in Richtung von dem blinden Ende 60 zu dem offenen Ende hin, wie durch einen Pfeil 68 in Fig. 5 angedeutet ist.

Im Falle einer rein kapillaren Befüllung ist der Übergangsbereich 62 derart ausgestaltet, dass der kapillare Fluss dort nicht unterbrochen wird. Eine wichtige Maßnahme hierzu stellt beispielsweise die Vermeidung scharfer Übergangskanten dar. Wird diese Endphase der kapillaren Befüllung durch eine Zentrifugation unterstützt, so sind im Bereich 62 auch nicht rein kapillar befüllbare Geometrien tolerierbar, ohne die Gesamtfunktionalität der Blindkanalbasierten Hämatokritbestimmung zu gefährden.

Kanalstrukturen, beispielsweise eine solche, wie sie in Fig. 5 gezeigt ist, können beispielsweise unter Verwendung einer CNC-Mikromaterialbearbeitung (CNC = computer numerically controlled) in eine COC-Scheibe (COC = Cyclic Olefin Copolymer) unter Verwendung eines sich verjüngenden Werkzeugs erzeugt werden, was Wände mit einer Neigung von 17,5° ergibt. Die obere und die untere Ebene der in Fig. 5 gezeigten Zwei-Ebenen-Kapillarstruktur können beispielsweise eine Tiefe von 400 µm, Breiten von 1.400 µm bzw. 400 µm und radiale Längen von 25 mm bzw. 25,4 mm aufweisen, mit einem Übergang am geschlossenen Ende 60, wie oben erläutert wurde.

Die inneren Kanalwände sind nach dem Erzeugen des Kanals aufgrund des verwendeten Substratmaterials bezüglich der zu untersuchenden Suspension hydrophilisiert oder werden nach dem Erzeugen der Kanalstrukturen entsprechend hydrophilisiert.

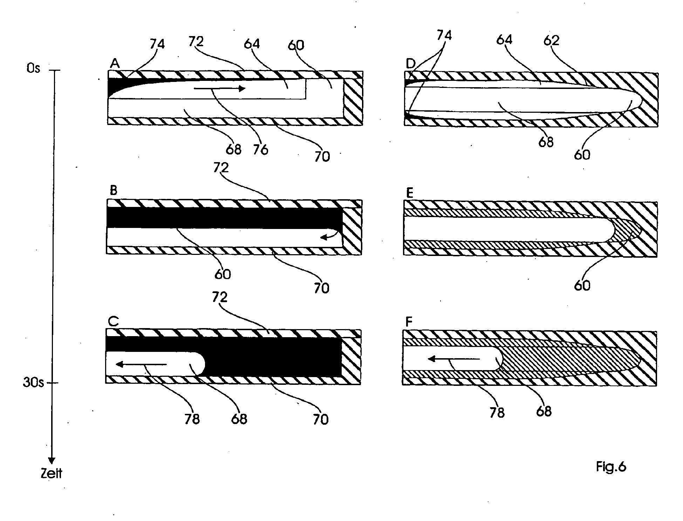

Eine Sequenz, die die Befüllung eines Blindkanals, wie er in den Fig. 3A und 5 gezeigt ist, darstellt, ist in den Fig. 6A - 6F gezeigt, wobei die Fig. 6A - 6C seitliche Längsschnittansichten zeigen, während die Fig. 6D - 6F Draufsichten auf die in Fig. 5 gezeigte Kanalstruktur darstellen. Die dargestellte Befüllung erfolgt ohne Zentrifugalkraftunterstützung, wobei eine Zeitachse am linken Rand der Fig. 6A bis 6C andeutet, dass der Befüllvorgang bis zu dem Befüllungsgrad, der in den Fig. 6C und 6F gezeigt ist, ca. 30 Sekunden dauert.

Wie in Fig. 6 zu erkennen ist, ist der Blindkanal 62 in ein Substrat 70 strukturiert und mittels eines Deckels 72 geschlossen. Der Kanal besitzt, wie Bezug nehmend auf die Fig. 4A und 5 erläutert wurde, Bereiche 64, in denen eine erhöhte Kapillarkraft herrscht, und Bereiche 68, in denen eine geringere Kapillarkraft herrscht.

Auf ein Einbringen einer Suspension in einen (in den Fig. 6A - 6F nicht gezeigten) Einlassbereich, der am offenen Ende mit dem Blindkanal 62 fluidisch verbunden ist, wird durch die Kapillarkraft die Suspension entlang der kritischen Kanten zwischen den geneigten Seitenwänden und dem Deckel gezogen, wie durch die Suspensionsbereiche 74 in den Fig. 6A und 6D gezeigt und durch den Pfeil 76 in Fig. 6A angedeutet ist. Nach dem Befüllen der Bereiche 64 in Richtung von dem offenen Ende zu dem geschlossenen Ende des Blindkanals 62, unterstützt die spezielle Form des geschlossenen Endes einen nahtlosen Übergang der Suspension in den Bereich 68 entlang der Ränder, wie in den Fig. 6B und 6E zu erkennen ist. Dieser Übergang in den Bereich 68 wird weiterhin dadurch unterstützt, dass die Ränder an dem geschlossenen Ende der Blindkapillare abgerundet sind. Anschließend erfolgt eine Auffüllung des noch ungefüllten Bereichs 68 in Richtung von dem geschlossenen Ende 60 des Blindkanals 62 zu dem offenen Ende desselben. Dies führt zu einer vollständigen Evakuierung des Kanals, sodass dieser im Wesentlichen ohne Blaseneinschluss vollständig durch die Suspension befüllt wurde.

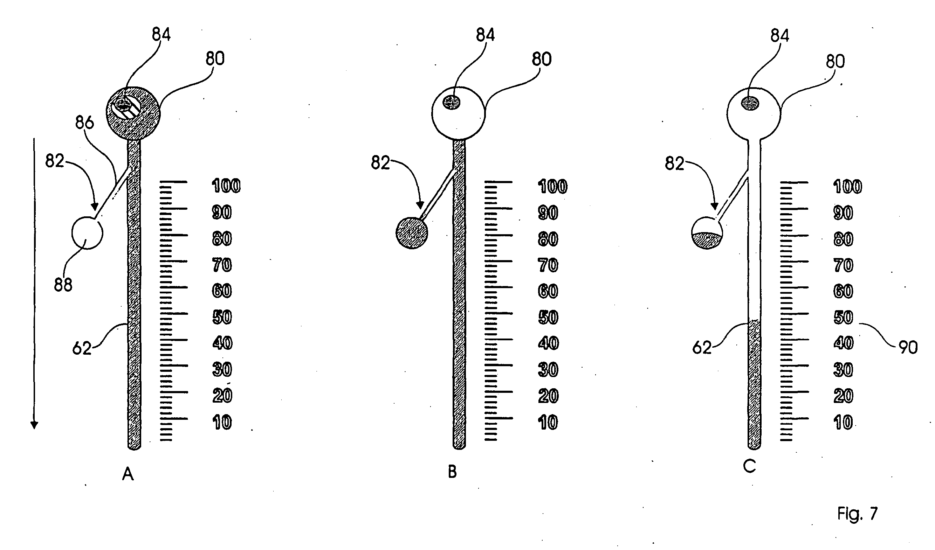

Eine Durchführung eines Beispiels eines erfindungsgemäßen Verfahrens unter Verwendung einer Kanalstruktur, die einen Kanal 62, wie er oben beschrieben wurde, aufweist, ist in den Fig. 7A - 7C gezeigt. Die Kanalstruktur umfasst den Blindkanal 62, einen Einlassbereich 80 sowie eine Überlaufstruktur 82. Die genannten Kanalstrukturen können wiederum in einem Substrat gebildet sein und durch einen Deckel abgedeckt sein, der wiederum eine Öffnung 84, zum Einbringen eine Suspension in den Einlassbereich 80, der ein Einlassreservoir darstellen kann, aufweisen kann.

In Fig. 7A ist der Zustand gezeigt, bei dem der Blindkanal 62 vollständig mit der zu sedimentierenden Suspension gefüllt ist. Nach dieser Befüllung wird die Drehfrequenz über die Durchbruchsfrequenz eines am Eingang hydrophobisierten Überlaufkanals 86 der Überlaufstruktur 82 erhöht, sodass die überschüssige Suspension über den Überlaufkanal 86 in das Überlaufreservoir 88 abgezogen wird. Fig. 7B zeigt die Kanalstruktur nach dem Abdosieren der überschüssigen Suspension unter Verwendung der Überlaufstruktur 82. Die Grenzfrequenz für den Durchbruch kann beispielsweise 30 Hz betragen, wobei das Suspensionsvolumen in der Blindkapillare 62 beispielsweise 20 µl betragen kann. Anschließend wird das Substrat, in dem die Kanalstruktur gebildet ist, weiterhin einer Rotation unterworfen, beispielsweise für fünf Minuten bei 100 Hz, so dass die in dem Blindkanal 62 befindliche Suspension sedimentiert wird. Fig. 7C zeigt die Kanalstruktur nach dem Sedimentieren. Der Volumenanteil des abgelagerten Sediments bzw. der Hämatokritwert kann dann bei Ruhe durch das Verhältnis der radialen Position der Flüssigkeits-Festkörpergrenzfläche und die bekannte Länge der Kapillare bestimmt werden. Vorzugsweise kann hierbei eine auf dem Substrat befindliche Skala 90 zum Ablesen des Hämatokritwerts verwendet werden.

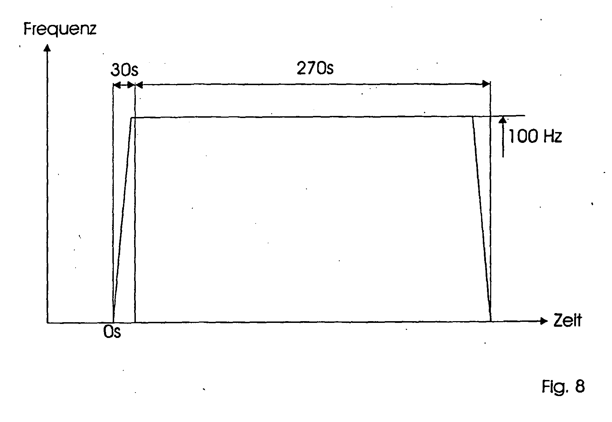

Fig. 8 zeigt ein mögliches Frequenzprotokoll zum Betreiben der Antriebsvorrichtung, beispielsweise des Drehmotors. Zu Beginn wird die Drehfrequenz auf beispielsweise 100 Hz hochgefahren, wobei die hierdurch erzeugte Zentrifugalkraft den Befüllvorgang unterstützen kann. Nach Überschreiten der Grenzfrequenz der Überlaufstruktur läuft die übermäßige Suspension in das Suspensionsreservoir 88. Um eine Sedimentation der Suspension in dem Blindkanal zu bewirken, findet eine Rotation bei einer im Wesentlichen konstanten Drehgeschwindigkeit statt, woraufhin die Rotation durch Abbremsen über einen bestimmten Zeitraum beendet wird. Nach dem Stillstand kann dann der Volumenanteil unter Verwendung der Skala durch einen Bediener oder automatisch über eine optische Erfassungseinrichtung abgelesen werden.

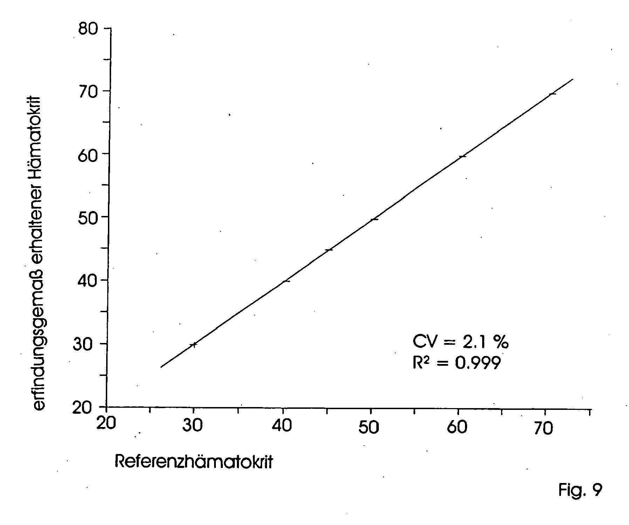

Fig. 9 zeigt das Ergebnis einer Messreihe zur Bestimmung des Hämatokritwertes, die unter Verwendung der oben beschriebenen Vorrichtung und mit dem beschriebenen Verfahren erhalten wurde. Die Referenzbestimmung erfolgt hier mit Hilfe eines Mikrohämatokrit-Rotors Z 233 M-2 von der Firma Hermle Labortechnik in einer Zentrifuge von der gleichen Firma.

Fig. 9 zeigt, dass ein CV-Wert von 2,1% und eine hohe Linearität zwischen dem erfindungsgemäß erhaltenen Hämatokritwert und der Referenzmessung, R² = 0,999, bei einer Bestimmungszeit von fünf bis sechs Minuten erhalten wurde.

Die vorliegende Erfindung schafft somit ein neuartiges Konzept, das zur Bestimmung eines zentrifugenbasierten Hämatokrittests in einer Blindkapillare geeignet ist. Der Test kann durch ein Frequenzprotokoll auf einer einfachen Zwei-Ebenen-Kanalstruktur implementiert werden, die ohne weiteres unter Verwendung einer kostengünstigen Massenherstellung, beispielsweise Spritzgießen, erreicht werden kann. Der Test ist sehr genau und erfordert ein Blutvolumen von nur 20 µl. Darüber hinaus macht das Auslesen durch eine visuelle Inspektion auf einer aufgedruckten Skala den Bedarf nach einer aufwendigen Erfassungsausrüstung überflüssig, wobei der Hämatokrittest prinzipiell auf einem herkömmlichen CD-Laufwerk ablaufen könnte. Um eine Rotationssymmetrie der Scheibe zu erreichen, kann es ferner vorteilhaft sein, eine Parallelisierung von Kanälen zu implementieren, wie oben Bezug nehmend auf Fig. 10B erläutert wurde, was von besonderem Vorteil für eine routinemäßige Bluttrennung ist.

Bei Ausführungsbeispielen der vorliegenden Erfindung kann ferner eine Möglichkeit vorgesehen sein, um ein Auslesen während oder nach der Rotation zu ermöglichen. Zu diesem Zweck kann ein geeignetes Messinstrument vorgesehen sein. Dieses kann beispielsweise eine Fotokamera mit kurzer Öffnungszeit oder eine stroboskopische Kamera aufweisen, um den Blindkanal, gegebenenfalls mit einer zugeordneten Skala, zu erfassen. Das Messinstrument kann ferner eine Auswertungseinrichtung aufweisen, um die erfassten Bilder auszuwerten und daraus den Hämatokritwert zu bestimmen.

Das Substrat, in welchem die Kanalstrukturen gebildet sind, kann aus beliebigen geeigneten Materialien gebildet sein, beispielsweise Kunststoff, Silizium, Metall oder dergleichen. Ferner können das Substrat und die darin gebildeten Strukturen durch geeignete Herstellungsverfahren erzeugt werden, beispielsweise Mikrostrukturierung oder Spritzgusstechniken. Der Deckel des erfindungsgemäßen Substrats kann aus einem geeigneten, vorzugsweise durchsichtigen Material, beispielsweise Glas oder Pyrexglas, bestehen.

Bezug nehmend auf die bevorzugten Ausführungsbeispiele wurde der Körper aus Substrat und Deckel als ein Rotationskörper mit einer Rotationsachse beschrieben, wobei die Antriebseinrichtung ausgebildet ist, um den Rotationskörper um seine Rotationsachse zu drehen. Alternativ kann der Körper eine im Wesentlichen beliebige Form aufweisen, wobei die Antriebseinrichtung eine Halterung zum Halten des Körpers und zum Drehen des Substrats um eine außerhalb des Substrats liegende Rotationsachse aufweist.

## Patentansprüche

1. Vorrichtung zur Bestimmung der Volumenanteile der Phasen in einer Suspension mit folgenden Merkmalen:
einem Körper (18);
einer Kanalstruktur, die in dem Körper (18) gebildet ist und einen Einlassbereich (30; 80) und einen Blindkanal (32; 62), der mit dem Einlassbereich (30; 80) fluidisch verbunden und über denselben befüllbar ist, aufweist; und
einer Antriebseinrichtung (22, 24, 26) zum Beaufschlagen des Körpers (18) mit einer Rotation, sodass durch eine Zentrifugation eine Phasentrennung der Suspension in dem Blindkanal (32; 62) stattfindet,
wobei der Blindkanal (32, 62) einen solchen Kanalquerschnitt und/oder solche Benetzungseigenschaften aufweist, dass beim Befüllen desselben über den Einlassbereich (30; 80) mit der Suspension in einem ersten Querschnittbereich (64) höhere Kapillarkräfte als in einem zweiten Querschnittbereich (68) wirken, sodass sich zunächst der erste Querschnittbereich (64) in Richtung von dem Einlassbereich (30; 80) zu dem blinden Ende (60) des Blindkanals (32; 62) hin füllt und dann der zweite Querschnittbereich (68) in Richtung von dem blinden Ende (60) zu dem Einlassbereich (30; 80) hin füllt.

2. Vorrichtung nach Anspruch 1, bei der die Kanalstruktur ferner eine Überlaufstruktur (36; 82) zwischen dem Einlassbereich (30; 80) und dem Blindkanal (32; 62) zur Volumendosierung der Suspension aufweist.

3. Vorrichtung nach Anspruch 1 oder 2, bei der der Körper (18) eine Skala (42) aufweist, die relativ zu dem Blindkanal (32; 62) derart angeordnet ist, dass der Volumenanteil im Blindkanal (32; 62) ablesbar ist.

4. Vorrichtung nach einem der Ansprüche 1 bis 3, bei der der Körper (18) eine erste Schicht (12; 70), in der die Kanalstruktur gebildet ist, und eine zweite Schicht (16; 72), die einen Deckel bildet, aufweist.

5. Vorrichtung nach einem der Ansprüche 1 bis 4, bei der der Körper (18) als ein Rotationskörper mit eine Rotationsachse ausgebildet ist, wobei die Antriebseinrichtung (22, 24, 26) ausgebildet ist, um den Rotationskörper um seine Rotationsachse zu drehen.

6. Vorrichtung nach einem der Ansprüche 1 bis 4, bei der die Antriebseinrichtung eine Halterung zum Halten des Körpers und zum Drehen des Körpers um eine außerhalb des Körpers liegende Rotationsachse aufweist.

7. Vorrichtung nach einem der Ansprüche 1 bis 6, bei der der Blindkanal Wände aufweist, die mit unterschiedlichen, zwischen denselben eingeschlossenen Winkeln aneinandergrenzen.

8. Vorrichtung nach einem der Ansprüche 1 bis 7, bei der der Blindkanal Wände aufweist, die bezüglich der Suspension unterschiedlich hydrophil sind oder die Abschnitte aufweisen, die bezüglich der Suspension unterschiedlich hydrophil sind.

9. Vorrichtung nach einem der Ansprüche 1 bis 8, bei der der Blindkanal (32; 62) einen Querschnitt mit zumindest einer Stufe aufweist.

10. Vorrichtung nach einem der Ansprüche 1 bis 9, bei der der Blindkanal eine T-förmige Kanalgeometrie aufweist.

11. Vorrichtung nach einem der Ansprüche 1 bis 10, bei der der Blindkanal eine obere Wand und eine untere Wand und zumindest eine bezüglich der oberen Wand und der unteren Wand in einem von 90° verschiedenen Winkel angeordnete Seitenwand aufweist.

12. Vorrichtung nach einem der Ansprüche 1 bis 11, die ferner eine Einrichtung zur Bestimmung der Volumenanteile in dem Blindkanal während oder nach der Rotation aufweist.

13. Verfahren zum Bestimmen der Volumenanteile der Phasen in einer Suspension mit folgenden Schritten:
Bereitstellen einer Kanalstruktur, die einen Einlassbereich (30; 80) und einen an den Einlassbereich (30; 80) angrenzenden Blindkanal (32; 62) aufweist;
Einbringen der Suspension in den Einlassbereich (30; 80), wobei der Blindkanal (32; 62) einen solchen Kanalquerschnitt und/oder solche Benetzungseigenschaften aufweist, dass in einem ersten Querschnittbereich (64) höhere Kapillarkräfte als in einem zweiten Querschnittbereich (68) wirken, sodass sich zunächst der erste Querschnittbereich in Richtung von dem Einlassbereich (30; 80) zu dem blinden Ende (60) hin füllt und dann der zweite Querschnittbereich (68) in Richtung von dem blinden Ende (60) zu dem Einlassbereich (30; 80) hin füllt; und
Beaufschlagen der Kanalstruktur mit einer Rotation, um eine Phasentrennung der Suspension in dem Blindkanal (32; 62) durch Zentrifugation zu bewirken.

14. Verfahren nach Anspruch 13, bei dem die Kanalstruktur vor dem vollständigen Befüllen des Blindkanals (32; 62) mit einer Rotation beaufschlagt wird, um die Befüllung durch Ausnutzung einer Zentrifugalkraft zu beschleunigen.

15. Verfahren nach Anspruch 13 oder 14, bei dem die Suspension Blut ist und bei dem die Abmessungen der Kanalstruktur angepasst sind, um den Hämatokrit von Blut zu bestimmen.

16. Verfahren einem der Ansprüche 13 bis 15, das ferner einen Schritt des Bestimmens der Volumenanteile in dem Blindkanal während oder nach der Rotation aufweist.

## Claims

1. Apparatus for determining the volume fractions of the phases in a suspension, comprising:
a body (18);
a channel structure, which is formed in the body (18) and comprises an inlet area (30; 80) and a blind channel (32; 62), which is fluidically connected to and capable of being filled via the inlet area (30; 80); and
a drive (22, 24, 26) for imparting the body (18) with rotation, so that phase separation of the suspension in the blind channel (32; 62) takes place by centrifugation,
wherein the blind channel (32; 62) comprises such a channel cross-section and/or such wetting properties that, when filling same with the suspension via the inlet area (30; 80), higher capillary forces act in a first cross-sectional area (64) than in a second cross-sectional area (68), so that at first the first cross-sectional area (64) fills in the direction from the inlet area (30; 80) toward the blind end (60) of the blind channel (32; 62) and then the second cross-sectional area (68) fills in the direction from the blind end (60) toward the inlet area (30; 80).

2. Apparatus according to claim 1, wherein the channel structure further comprises an overflow structure (36; 82) between the inlet area (30; 80) and the blind channel (32; 62) for volume dosage of the suspension.

3. Apparatus according to claims 1 or 2, wherein the body (18) comprises a scale (42), which is arranged relative to the blind channel (32; 62) such that the volume fraction in the blind channel (32; 62) can be read.

4. Apparatus according to any of claims 1 to 3, wherein the body (18) comprises a first layer (12; 70), in which the channel structure is formed, and a second layer (16; 72), which forms a lid.

5. Apparatus according to any of claims 1 to 4, wherein the body (18) is formed as a rotation body with a rotation axis, wherein the drive (22, 24, 26) is formed to rotate the rotation body about its rotation axis.

6. Apparatus according to any of claims 1 to 4, wherein the drive comprises a fixture for holding the body and for rotating the body about a rotation axis lying outside the body.

7. Apparatus according to any of claims 1 to 6, wherein the blind channel comprises walls bordering on each other with different angles enclosed therebetween.

8. Apparatus according to any of claims 1 to 7, wherein the blind channel comprises walls, which are differently hydrophilic with respect to the suspension or which comprise portions being differently hydrophilic with respect to the suspension.

9. Apparatus according to any of claims 1 to 8, wherein the blind channel (32; 62) comprises a cross-section with at least one step.

10. Apparatus according to any of claims 1 to 9, wherein the blind channel comprises a T-shaped channel geometry.

11. Apparatus according to any of claims 1 to 10, wherein the blind channel comprises an upper wall and a lower wall and at least one sidewall arranged at an angle different from 90° with respect to the upper wall and the lower wall.

12. Apparatus according to any of claims 1 to 11, further comprising a determinator for determining the volume fractions in the blind channel during or after the rotation.

13. Method for determining the volume fractions of the phases in a suspension, comprising:
providing a channel structure, which comprises an inlet area (30; 80) and a blind channel (32; 62), which borders on the inlet area (30; 80);
introducing the suspension into the inlet area (30; 80), wherein the blind channel (32; 62) comprises such a channel cross-section and/or such wetting properties that higher capillary forces act in a first cross-sectional area (64) than in a second cross-sectional area (68), so that at first the first cross-sectional area fills in the direction from the inlet area (30; 80) toward the blind end (60) and then the second cross-sectional area (68) fills in the direction from the blind end (60) toward the inlet area (30; 80); and
imparting the channel structure with rotation, to cause phase separation of the suspension in the blind channel (32; 62) by centrifugation.

14. Method according to claim 13, wherein the channel structure is imparted with rotation prior to complete filling of the blind channel (32; 62), in order to accelerate the filling by taking advantage of centrifugal force.

15. Method according to claims 13 or 14, wherein the suspension is blood and wherein the dimensions of the channel structure are adapted to determine the hematocrit of blood.

16. The method according to any of claims 13 to 15, further comprising determining the volume fractions in the blind channel during or after the rotation.

## Revendications

1. Dispositif pour déterminer les fractions volumiques des phases dans une suspension, aux caractéristiques suivantes:
un corps (18);
une structure de canal formée dans le corps (18) et présentant une zone d'entrée (30; 80) et un canal borgne (32; 62) relié en connexion de fluide à la zone d'entrée (30; 80) et pouvant être rempli via cette dernière; et
un moyen d'entraînement (22, 24, 26) destiné à soumettre le corps (18) à une rotation, de sorte qu'ait lieu, par une centrifugation, une séparation de phases de la suspension dans le canal borgne (32; 62),
le canal borgne (32, 62) présentant une section de canal et/ou des propriétés d'humidification telles que lors du remplissage en suspension de celui-ci via la zone d'entrée (30; 80) agissent, dans une première zone de section (64) des forces capillaires plus grandes que dans une deuxième zone de section (68), de sorte que se remplisse tout d'abord la première zone de section (64) dans le sens de la zone d'entrée (30; 80) vers l'extrémité borgne (60) du canal borgne (32; 62) et que se remplisse ensuite la deuxième zone de section (68) dans le sens de l'extrémité borgne (60) vers la zone d'entrée (30; 80).

2. Dispositif selon la revendication 1, dans lequel la structure de canal présente par ailleurs une structure de surverse (36; 82) entre la zone d'entrée (30; 80) et le canal borgne (32; 62) destinée au dosage de volume de la suspension.

3. Dispositif selon la revendication 1 ou 2, dans lequel le corps (18) présente une graduation (42) qui est disposée par rapport au canal borgne (32; 62) de sorte que puisse être lue la fraction volumique dans le canal borgne (32; 62).

4. Dispositif selon l'une des revendications 1 à 3, dans lequel le corps (18) présente une première couche (12; 70), dans laquelle est formée la structure de canal, et une deuxième couche (16; 72) formant un couvercle.

5. Dispositif selon l'une des revendications 1 à 4, dans lequel le corps (18) se présente sous forme de corps rotatif avec un axe de rotation, le moyen d'entraînement (22, 24, 26) étant réalisé de manière à faire tourner le corps rotatif autour de son axe de rotation.

6. Dispositif selon l'une des revendications 1 à 4, dans lequel le moyen d'entraînement présente un support pour maintenir le corps et pour faire tourner le corps autour d'un axe de rotation se trouvant en dehors du corps.

7. Dispositif selon l'une des revendications 1 à 6, dans lequel le canal borgne présente des parois adjacentes l'une à l'autre selon des angles différents formés entre elles.

8. Dispositif selon l'une des revendications 1 à 7, dans lequel le canal borgne présente des parois hydrophiles à des degrés différents par rapport à la suspension ou présentant des segments qui sont hydrophiles à des degrés différents par rapport à la suspension.

9. Dispositif selon l'une des revendications 1 à 8, dans lequel le canal borgne (32; 62) présente une section avec au moins un étage.

10. Dispositif selon l'une des revendications 1 à 9, dans lequel le canal borgne présente une géométrie de canal en forme de T.

11. Dispositif selon l'une des revendications 1 à 10, dans lequel le canal borgne présente une paroi supérieure et une paroi inférieure et au moins une paroi latérale disposée selon un angle différant de 90° par rapport à la paroi supérieure et à la paroi inférieure.

12. Dispositif selon l'une des revendications 1 à 11, présentant par ailleurs un moyen pour déterminer les fractions volumiques dans le canal borgne pendant ou après la rotation.

13. Procédé pour déterminer les fractions volumiques des phases dans une suspension, aux étapes suivantes consistant à:
préparer une structure de canal présentant une zone d'entrée (30; 80) et un canal borgne (32; 62) adjacent à la zone d'entrée (30; 80);
introduire la suspension dans la zone d'entrée (30; 80), le canal borgne (32; 62) présentant une section de canal et/ou des propriétés d'humidification telles que dans une première zone de section (64) agissent des forces capillaires plus grandes que dans une deuxième zone de section (68), de sorte que se remplisse tout d'abord la première zone de section dans le sens de la zone d'entrée (30; 80) vers l'extrémité borgne (60) et que se remplisse ensuite la deuxième zone de section (68) dans le sens de l'extrémité borgne (60) vers la zone d'entrée (30; 80); et
soumettre la structure de canal à une rotation, pour provoquer par centrifugation une séparation de phases de la suspension dans le canal borgne (32; 62).

14. Procédé selon la revendication 13, dans lequel la structure de canal est, avant le remplissage complet du canal borgne (32; 62), soumise à une rotation, pour accélérer le remplissage en mettant à profit une force centrifuge.

15. Procédé selon la revendication 13 ou 14, dans lequel la suspension est du sang et dans lequel les dimensions de la structure de canal sont adaptées pour déterminer l'hématocrite du sang.

16. Procédé selon l'une des revendications 13 à 15, présentant par ailleurs une étape consistant à déterminer les fractions volumiques dans le canal borgne pendant ou après la rotation.
